# EUROPEAN PATENT SPECIFICATION

(11) **EP 3 484 294 B1**
(45) Date of publication and mention of the grant of the patent: **19.05.2021**
(21) Application number: 17742841.4
(22) Date of filing: 17.07.2017
(51) Int. Cl.: A01N 47/46, A01N 63/30, A01N 65/08, C09K 17/00, A01P 5/00

(54) **BIOFUMIGANT**
BIOBEGASUNGSMITTEL
BIOFUMIGANT

(30) Priority: 18.07.2016 GB 201612424; 17.11.2016 GB 201619489
(43) Date of publication of application: 22.05.2019
(73) Proprietor: Barworth Agriculture Ltd, Sleaford, NG34 9NB (GB)
(72) Inventor: BARKER, Anthony, Heckington Sleaford NG34 9RP (GB)
(74) Representative: Appleyard Lees IP LLP
(86) International application number: PCT/GB2017/052100
(87) International publication number: WO 2018/015729

(56) References cited:
- WO-A1-2014/205550
- WO-A1-2016/038365
- WO-A2-2012/037352
- CN-B- 102 604 842
- FRANZISKA S. HANSCHEN ET AL: "Degradation of Biofumigant Isothiocyanates and Allyl Glucosinolate in Soil and Their Effects on the Microbial Community Composition", PLOS ONE, vol. 10, no. 7, 17 July 2015 (2015-07-17), page e0132931, XP055407178, DOI: 10.1371/journal.pone.0132931
- NAOKI TANI ET AL: "Isolation of Myrosinase Producing Microorganism", AGRICULTURAL AND BIOLOGICAL CHEMISTRY, vol. 38, no. 9, 1 January 1974 (1974-01-01), pages 1617-1622, XP055219736, DOI: 10.1080/00021369.1974.10861387

## Description

The present invention relates to the field of biofumigants, more specifically the field of biofumigants for targeting crop diseases in solanacae species, for example, tomatoes and potatoes. In particular the present invention relates to biofumigants for the treatment of soil borne pathogens in agricultural and horticultural crops.

An example of soil borne pathogens are Plant Parasitic Nematodes, PPN. PPN are a pest that cause disease of the roots in many crop species. The problem leads to wide scale crop failure every year, with a significant economic impact. Current methods of tackling PPN are expensive, damaging to the environment, and have become increasingly ineffective.

Traditional pesticides such as fosthiazate are currently the standard treatment for addressing soil borne pathogens such as PPN. However application of fosthiazate and similar pesticides for bacteria and fungi borne pathogens is strictly controlled and the effectiveness is very dependent on soil conditions. Under some conditions, for example high temperature or higher soil pH, the pesticide can be degraded in as little as under ten days. Furthermore, such pesticides are likely to become obsolete in the near future due to the ever stricter regulations on the application of chemicals to soils and to plant products intended for consumption.

Alternative chemical solutions have been suggested. One example is the application of biocides to effectively disinfect soil of any microorganisms. However, although this is effective in the short term, only the top soil is cleared of microorganisms. In the longer term, microorganisms from the deeper layers of soil migrate up into the top soil and re-establish communities. Typically, it is the pathogenic microorganisms that establish the communities first, rendering the top soil more infective than before the biocide was applied.

The use of biological solutions has increased in recent years. One such solution is biofumigation. Biofumigation typically involves the generation of a natural biocide/pesticide from a sacrificial crop which is then macerated into the soil to release the biocide/pesticide and prepare the soil for the growth of the intended crop. For most soil pathogens including PPN, isothiocyanate compounds are effective biofumigants. The precursor chemicals glucosinolates are naturally produced by a series of plants together with myrosinase enzymes as a defence mechanism. When these plants are eaten or otherwise crushed, the cells release the glucosinolates and myrosinases, which then combine such that the myrosinases catalyse the conversion of glucosinolates into isothiocyanates.

However, a disadvantage of these biofumigation methods is that significant time and money is invested to plant a sacrificial crop which provides no revenue and is incorporated into a field before the desired crop can be grown. Furthermore, like any other chemical added to soil, the effectiveness of the naturally released isothiocyanates diminishes after a number of days depending on the weather conditions.

In WO2016038365, the applicant described treating soil with a glucosinolate- secreting plant or a source thereof and contacting said soil with a myrosinase-secreting microorganism or a source thereof.

WO2014205550 describes a pesticide formulation comprising a first part comprising a glucosinolate concentrate, and a second part comprising a plant material comprising myrosinase.

CN102604842 describes a trichoderma atroviride strain for producing myrosase.

FRANZISKA S. HANSCHEN ET AL, "Degradation of Biofumigant Isothiocyanates and Allyl Glucosinolate in Soil and Their Effects on the Microbial Community Composition", PLOS ONE, (20150717), vol. 10, no. 7 describes the use of brassicales species rich in glucosinolates for biofumigation.

WO2012037352 describes compositions for reducing pathogens in soil comprising intracellular components of lysed, beneficial, crop and non-crop rhizosphere-inhabiting yeast cells and whole or lysed, beneficial, crop and non-crop rhizosphere-inhabiting bacteria cells.

NAOKI TANI ET AL, "Isolation of Myrosinase Producing Microorganism", AGRICULTURAL AND BIOLOGICAL CHEMISTRY, (19740101), vol. 38, no. 9, doi:10.1080/00021369.1974.10861387, pages 1617 - 1622 describes screening tests for obtaining microorganisms which produce myrosinase.

It is an object of at least one or more aspects of the present invention to address one or more of the above-mentioned disadvantages in the prior art.

It is a further object of the invention to provide an alternative source of myrosinase.

According to a first aspect of the present invention, there is provided a method of treating soil, the method comprising providing the soil with a glucosinolate-secreting plant or a seed thereof and contacting said soil with a myrosinase-secreting yeast of the *Cryptococcous genus* or a spore thereof.

According to a second aspect of the present invention, there is provided the use of a combination of a myrosinase-secreting *Cryptococcus* yeast or a spore thereof, and a glucosinolate-secreting plant or a seed thereof for treating soil.

According to a third aspect of the present invention, there is provided a kit for treating soil, the kit comprising a glucosinolate-secreting plant or a seed thereof, and a myrosinase-secreting *Cryptococcous* yeast or a spore thereof.

Disclosed herein is the use of a yeast of the *Cryptococcus* genus to provide myrosinase.

Preferred features of the first, second, third and further aspects of the invention will now be described. Any feature of any aspect may apply to any other aspect as appropriate.

In the method of the first aspect of the present invention the soil treated is provided with a glucosinolate and a myrosinase. These suitably interact to produce an isothiocyanate. This isothiocyanate is suitably the active biocidal/pesticidal species, i.e. the biofumigant.

Advantageously, the use of a plant-yeast system to generate the biofumigant instead of destroying the natural cellular plant system means that the plants themselves do not have to be destroyed by macerating or incorporation into the field in order to provide the soil with biofumigant. Since the plant secretes the precursor chemical glucosinolates, external yeasts can provide the enzyme myrosinases to convert the precursors to the biofumigant outside the plant. This allows the farmer to use the biofumigant crop as a product and potentially generate revenue therefrom in addition to the increased revenue from the now healthy intended crop. Furthermore, the biofumigant crop can be grown together with the intended crop by intercropping or other such techniques, thereby generating the biofumigant in the soil continuously throughout the growing period of the intended crop. This avoids any tapering off of the biofumigant levels and maintains the soil at a certain level of biofumigant which avoids the return of any pests or disease, and allows the farmer more flexibility due to less dependency on soil conditions and weather.

The method of the first aspect of the present invention is suitably a biofumigation method.

Suitably, the biofumigant is an isothiocyanate. The isothiocyanate produced will typically depend on the glucosinolate secreted from the plant and the particular myrosinase enzyme secreted from the *Cryptococcus* yeast. The specific glucosinolate product will usually depend on the particular species of plant. The myrosinase produced will depend on the specific species of microorganism.

The method of the present invention relies on the presence of myrosinase and glucosinolates in the soil. If a soil is already rich in one of these components the invention may involve introducing the other component into the soil.

According to a further aspect of the present invention, there is provided the use of a myrosinase-secreting yeast of the *Cryptococcus genus* for treating soil wherein the soil comprises a source of glucosinolates.

Disclosed herein is the use of a glucosinolate-secreting plant for treating soil wherein the soil comprises a yeast of the *Cryptococcus genus* which secretes myrosinases.

The present invention suitably provides an isothiocyanate in the soil. In some preferred embodiments the isothiocyanate is selected from one or more optionally substituted aryl, allyl or alkyl isothiocyanate, more preferably a C1-C10 alkyl, allyl or aryl isothiocyanate, most preferably, benzyl isothiocyanate, isopropyl isothiocyanate, methyl isothiocyanate, 1-napthyl isothiocyanate, 2- phenylethyl isothiocyanate, phenyl isothiocyanate, propyl isothiocyanate or similar.

Optionally, the aspects of the invention may comprise more than one plant, and/or more than one *Cryptococcus* yeast. Accordingly, any references herein to the plant, or a *Cryptococcus* yeast include one or more plants and one or more yeasts.

The present invention involves a glucosinolate-secreting plant or a seed thereof. This may suitably be a plant or a seed. Preferably the plant is a crop plant or a seed thereof. Any references to a plant herein include the seed thereof as appropriate.

Preferably the plant is of the order Brassicales. Preferably the plant is of the family Brassicaceae. Still more preferably the plant is of the genera Brassica or Raphanus. More preferably the plant is selected from one of the following species *Brassica balearica, Brassica carinata, Brassica elongata, Brassica fruticulosa, Brassica hilarionis, Brassica juncea, Brassica napus, Brassica narinosa, Brassica nigra, Brassica oleracea, Brassica perviridis, Brassica rupestris, Brassica rapa, Brassica septiceps, Brassica tournefortii, Raphanus sativa, Raphanus caudatus, Raphanus confusus or Raphanus raphanistrum.* Still more Preferably the plant is of the species *Brassica oleracea, Brassica juncea, Brassica alba,* or *Raphanus sativus.* Most preferably the plant is of the species *Brassica alba, Brassica juncea, or Raphanus sativus.* Preferably the glucosinolate secreted from the plant is selected from one of the following: gluconasturtin, glucobrassicin, glucoalyssin, glucoraphanin, progoitrin, or sinigrin. More preferably the glucosinolate is sinigrin.

The method of the first aspect of the invention involves providing the soil with a glucosinolate secreting plant.

Suitably, providing the soil with a glucosinolate-secreting plant may include growing said plant in the soil, or distributing the seed of said plant in or on the soil by such processes as planting, spreading or drilling etc.

Optionally, the method may further comprise the step of inducing the secretion of glucosinolate from the plant and/or inducing the secretion of myrosinase from the *Cryptococcus* yeast. Optionally this inducement may comprise contacting the plant and/or *Cryptococcus* yeast with an inducing chemical such as an inducing hormone or pheromone or metabolic agent, for example the application of the messenger protein 'harpin' or the application of methyl jasmonate to the foliage. Alternatively, the inducement may comprise causing physical damage to plant cells, stimulation of the immune system or other methods of causing the plant stress.

The present invention involves contracting the soil with a myrosinase-secreting yeast of the *Cryptococcus genus* or a spore thereof.

The source of myrosinases may be a *Cryptococcus* yeast or a spore thereof. Preferably the yeast is a soil borne *Cryptococcus* yeast or a spore thereof. Accordingly, any references to a *Cryptococcus* herein include the spores thereof.

The yeast used in the present invention is of the *Cryptococcus genus.* Preferably the yeast is selected from one of the following species: *Cryptococcus neoformans, Cryptococcus gatti, Cryptococcus albidus, Cryptococcus uniguttulatus, Cryptococcus macerans, Cryptococcus oierensis, Cryptococcus adeliensis, Cryptococcus aerius, Cryptococcus albidosimilis, Cryptococcus antarcticus, Cryptococcus aquaticus, Cryptococcus ater, Cryptococcus bhutanensis, Cryptococcus consortionis, Cryptococcus curvatus, Cryptococcus phenolicus, Cryptococcus skinneri, Cryptococcus terreus and Cryptococcus vishniacci .*

Preferably the mryosinase-secreting yeast is selected from *Cryptococcus macerans, Cryptococcus oierensis* and mixtures thereof. In some embodiments the myrosinase-secreting yeast comprises *Cryptococcus macerans.* In some embodiments the myrosinase-secreting yeast comprises *Cryptococcus oierensis.*

The present inventors have isolated two particular strains of yeast which are effective at secreting myrosinase and can be used in the present invention. These are identified in SEQUENCE ID 1 and SEQUENCE ID 2 filed herewith.

In some embodiments the myrosinase secreting yeast has the SEQUENCE ID 1.

In some embodiments the myrosinase secreting yeast has the SEQUENCE ID 2.

Suitably the amount of myrosinase-secreting *Cryptococcus* yeast contacted with the soil is an amount necessary to produce the effective amount of myrosinase relative to the amount of glucosinolate secreted by the plants.

The method of the first aspect of the invention involves contacting the soil with a myrosinase-secreting *Cryptococcus* yeast.

Suitably, contacting the soil with a myrosinase-secreting yeast may include spreading said yeast on the soil, or depositing said yeast in the soil by such processes as spraying, ploughing or any other methods of incorporating into the soil. Preferably contacting the soil with a myrosinase-secreting yeast includes contacting the soil in the vicinity of a glucosinolate-secreting plant, more preferably in the vicinity of a root of a glucosinolate-secreting plant, still more preferably in the rhizosphere and/or rhizoplane of a root of a glucosinolate-secreting plant, most preferably on the root of a glucosinolate-secreting plant.

In one embodiment the *Cryptococcus* yeast may be coated onto a seed of the plant.

Thus, according to a seventh aspect of the present invention, there is provided a seed of a glucosinolate-secreting plant comprising a coating, wherein the coating comprises a myrosinase-secreting *Cryptococcus* yeast or a spore thereof.

Preferably the seed comprises a coating in which the myrosinase-secreting yeast is trapped or encased. Preferably the coating is formed from an aqueous solution in which the microorganism is suitably suspended. Preferably the aqueous solution comprises one or more polymers. Preferably the polymers are adhesion promoting polymers such as, for example, carboxymethylcellulose (CMC).

Suitably, the coating is applied to the seed by spraying, dipping, immersion, painting or any other appropriate method.

In one embodiment, the present invention relates to one plant and one *Cryptococcus* yeast, which suitably each secrete one glucosinolate and one myrosinase respectively. Preferably the plant is *Brassica oleracae,* more preferably a cultivar of Purple Sprouting broccoli (Redhead), and the yeast is selected from *Cryptococcus macerans* and *Cryptococcus oierensis.* Preferably the glucosinolate is an alkyl glucosinolate. Examples of alkyl glucosinolates include sinigrin. Advantageously, purple sprouting broccoli is a high value crop in itself that can be grown for the purposes of revenue and also for the purposes of soil treatment according to the present invention, effectively performing both functions at once. Advantageously, *Cryptococcus macerans* and *Cryptococcus oierensis* are yeasts which are easy to culture in bulk in a laboratory environment and which grows naturally in soil. Therefore the yeasts can be produced easily and has survive well when transferred into the soil for the treatment.

Alternatively, other pairings of plants and yeasts, or glucosinolates and myrosinases may be appropriate. Other suitable embodiments of one plant and one microorganism include: *Sinapsis alba* and *Cryptococcus macerans; Sinapsis* alba and *Cryptococcus oierensis;* and *Brassica juncea* and *Cryptococcus macerans;* and *Brassica juncea* and *Cryptococcus oierensis.*

Preferably the soil treatment is for the benefit of crop plants, more preferably for crop plants at risk of loss from soil borne pathogens, still more preferably crops of the order Solanales, still more preferably for crop plants of the family Solanaceae, preferably for crop plants from the genus Solanum. Most preferably the soil treatment is for crop plants selected from the following species *Solanum tuberosum* and/or *Solanum lycopersicum.*

Thus the present invention may provide a method of improving the yield of a crop plant, the method comprising treating soil in which the crop plant is to be grown according to the method of the first aspect.

Suitably the method of the present invention provides a method of combatting a pathogenic infection in the soil. Suitably the method combats an infection of one or more pathogenic microorganisms, preferably pathogenic nematodes, more preferably cyst nematodes, most preferably potato cyst nematodes of the genus Globodera, for example the species *Globodera rostochiensis* and *Globodera pallida.*

By combatting pathogens we mean to refer to killing some or all of the pathogens present in the soil, or preventing the growth of the number of pathogens in the soil, reducing infection, reducing activity of the pest or acting as an antifeedant and/or repellent towards the pathogen. Suitably combatting pathogens reduces the number of pathogens in the soil.

Preferably the treatment method of the first aspect reduces the concentration of pathogens in the soil. Suitably it reduces the concentration of pathogenic microorganisms in the soil. Preferably it reduces the concentration of pathogenic nematodes in the soil. More preferably it reduces the concentration of potato cyst nematodes in the soil.

Preferably the treatment of the soil reduces the concentration of pathogenic microorganisms in the soil by at least 5%, preferably by at least 10%, suitably at least 15%, more preferably by at least 20%, still more preferably 25%. It may reduce the concentration of the pathogenic microorganisms in the soil by at least 30%, suitably at least 50%, for example at least 60%, at least 70%, or at least 80%.

Preferably the treatment of the soil reduces the concentration of pathogenic microorganisms in the soil to below the commercial economic damage threshold.

The reduction of pathogens using traditional biofumigation methods in which a sacrificial crop is macerated in to the soil may be from 10% to 70%. The present invention provides a reduction in pathogens within this range. In some embodiments the method of the present invention also provides the glucosinolate-secreting plant as a commercially viable crop. However even when intended as a sacrificial crop, the present invention provides advantages over the prior art. This is because the biofumigant is produced in the soil during the growing phase of the glucosinolate-secreting plant. Unlike traditional biofumigation methods in which the crop is mulched into the soil, there is no need to wait until at least 50% of the crop has flowered.

The invention will now be further explained by way of example only with reference to the following examples and accompanying figures in which:

### Example 1

The effectiveness of various microorganisms at degrading sinigrin was tested. The tested organisms included different types of bacteria and fungi, including yeasts. Sinigrin is a glucosinolate expressed by some plants of the *Brassicaceace* family.

The tests were carried out using sinigrin-barium agar plates.

Microorganisms were tested in 5 mM sinigrin media, both with and without glucose. This was to investigate whether myrosinase secretion would be maintained in the presence of another carbon source.

The sinigrin concentration was measured after 1 day using a spectrophotometer @ 227.5 nm.

The most effective organisms were identified by DNA extraction and next generation sequencing using known techniques.

The two most promising species were found to be yeasts and their genomes are provided in the attached sequence listings. These are believed to be novel species and have been deposited with the National Collection of Yeast Cultures.

SEQUENCE ID 1 is believed to be a yeast of the *Cryptococcus macerans* species.

SEQUENCE ID 2 is believed to be a yeast of the *Cryptococcus oierenis* species.

### Example 2

The effectiveness at degrading sinigrin of the yeasts of Sequence ID 1 and Sequence ID 2 is shown in table 1, compared with the effectiveness of some bacteria and fungi.

**Table 1**

| Temperature (°C) | pH | Glucose present? | Microorganism | | | |
|---|---|---|---|---|---|---|
| | | | *Pseudomonas* | *Paecilomyces* | SEQ ID 1 | SEQ ID 2 |
| | | | mM singrin degraded per day | | | |
| 4 | 7 | Y | 0.002119 | 0.0059233 | 0.068774 | 0.050274 |
| 10 | 7 | Y | | 0.03737 | 0.0428455 | 0.069025 |
| 10 | 7 | N | | 0.0366505 | 0.0782975 | 0.072742 |
| 20 | 7 | Y | 0.020295 | 0.021389 | 0.130728 | 0.309697 |
| 20 | 7 | N | 0.026905 | 0.041608 | 0.195898 | 0.141093 |
| 4 | 5.8 | Y | 0.026156 | 0.011059 | 0.075355 | 0.105971 |
| 10 | 5.8 | Y | | 0.0383695 | 0.1166665 | 0.116347 |
| 10 | 5.8 | N | | 0.013629 | 0.1508395 | 0.078537 |
| 20 | 5.8 | Y | 0.01432 | 0.0269786 | 0.21814 | 0.140887 |
| 20 | 5.8 | N | 0.036319 | 0.070273 | 0.253426 | 0.199889 |
| 4 | 8 | Y | | 0.01219 | 0.1306555 | 0.0796165 |
| 10 | 8 | Y | | 0.005955 | 0.1333335 | 0.126299 |
| 20 | 8 | Y | | 0.02494 | 0.3205435 | 0.123701 |
| 20 | 8 | N | | 0.056155 | 0.276512 | 0.155096 |

### Example 3

Tests were carried out to check that seeds coated with the *Cryptococcus* yeasts of the invention were able to germinate.

Seeds were sprayed twice with an inoculm comprising a yeast of SEQUENCE ID 1 or SEQUENCE ID 2.

Once dried seeds were sown in 50 ml falcon tubes containing 35 ml of John Innes No. 2 compost and stored at 20°C.

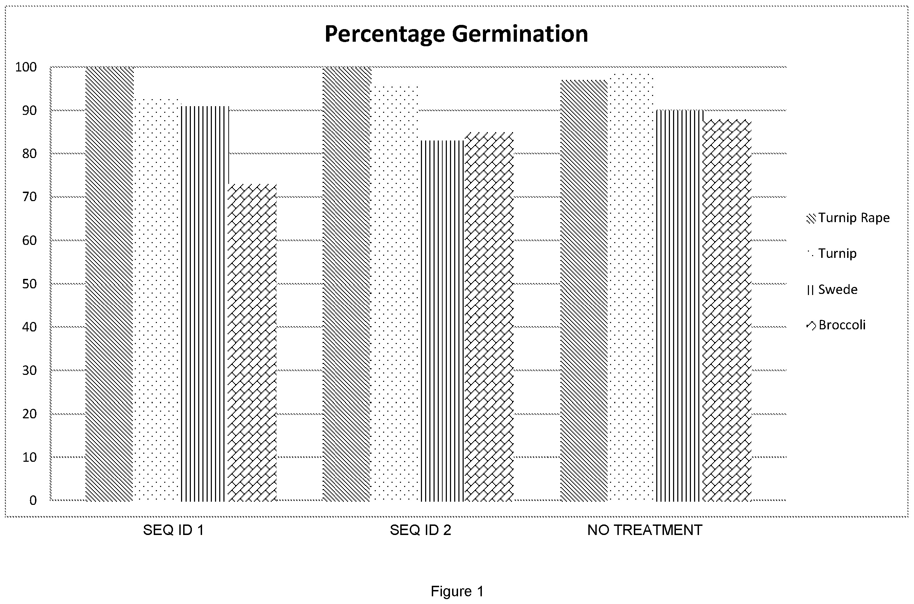

The percentage of seeds that had germinated was counted after 10 days. The results are shown in Figure 1.

### Example 4

Molecular analysis experiments were carried out to determine the presence of yeasts of SEQ ID1 and SEQ ID2.

Roots from 4 seed treated *Brassicas* were extracted from soil and stored in ethanol.

The presence of the yeasts of SEQ ID1 and SEQ ID2 was confirmed using Real-Time PCR protocols.

### Example 5

Root-Microbe interactions were studied by the following method.

Roots of plants grown from seeds treated with yeasts of SEQ ID 1 and SEQ ID 2 were checked for presence of these yeasts after 10 days of growth.

The roots were plated onto agar plates to examine growth of microbes.

The presence or absence of microbes was determined and the microbes identified using colony morophologies and real time PCR for SEQ ID1 and SEQ ID2.

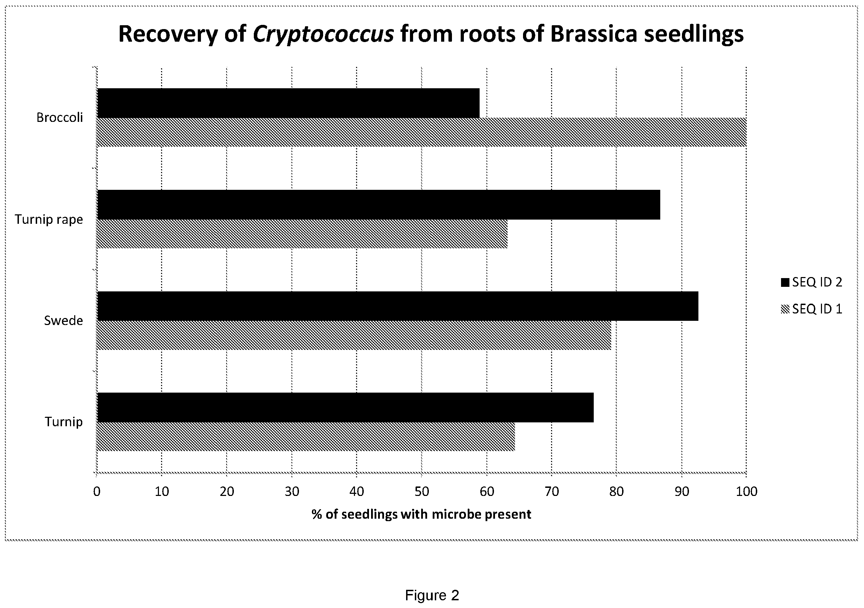

Figure 2 shows the percentage of the roots of different types of Brassica seedlings which had the yeasts present.

### Biological Deposits

The application refers to the following indications of deposited biological material:

| | |
|---|---|
| Name: | National Collection of Yeast Cultures |
| Address: | National Collection of Yeast Cultures, |
| | Institute of Food Research, |
| | Norwich Research Park, |
| | Norwich, |
| | Norfolk, |
| | NR4 7UA, |
| | United Kingdom |
| Date: | 10 August 2016 |
| Accession Number: | NCYC 4134, NCYC 4135 |
| Depositor: | Arcis Biotechnology |

## Claims

1. A method of treating soil, the method comprising providing the soil with a glucosinolate-secreting plant or a seed thereof and contacting said soil with a myrosinase-secreting yeast of the *Cryptococcus genus* or a spore thereof.

2. A method according to claim 1 which is a biofumigation method.

3. A method according to claim 1 or claim 2 wherein the glucosinolate and myrosinase interact to form an isothiocyanate.

4. A method according to claim 3 wherein the isothiocyanate is selected from benzyl isothiocyanate, isopropyl isothiocyanate, methyl isothiocyanate, 1-napthyl isothiocyanate, 2- phenylethyl isothiocyanate, phenyl isothiocyanate and propyl isothiocyanate.

5. A method according to any preceding claim wherein the glucosinolate-secreting plant is of the order Brassicales.

6. A method according to any preceding claim which is a method of combating a pathogenic infection in the soil.

7. A method according to claim 6 which is a method of combatting pathogenic Nematodes.

8. The use of a combination of a myrosinase-secreting *Cryptococcus* yeast or a spore thereof, and a glucosinolate-secreting plant or a seed thereof for treating soil.

9. A kit for treating soil, the kit comprising a glucosinolate-secreting plant or a seed thereof, and a myrosinase-secreting *Cryptococcus* yeast or a spore thereof.

10. The use of a myrosinase-secreting *Cryptococcus* yeast for treating soil wherein the soil comprises a source of glucosinolates.

11. A seed of a glucosinolate-secreting plant comprising a coating, wherein the coating comprises a myrosinase-secreting *Cryptococcus* yeast or a source thereof.

12. A method, use, kit or seed according to any preceding claim wherein the myrosinase-secreting yeast is selected from *Cryptococcus macerans* and *Cryptococcus oierensis.*

13. A method, use, kit or seed according to any preceding claim wherein the myrosinase-secreting yeast has the SEQUENCE ID 1 or the SEQUENCE ID 2.

## Patentansprüche

1. Verfahren zur Erdbodenbehandlung, wobei man bei dem Verfahren dem Erdboden eine Glucosinolat sezernierende Pflanze oder einen Samen davon bereitstellt und den Erdboden mit einer Myrosinase sezernierenden Hefe der *Cryptococcus-Gattung* oder einer Spore davon in Kontakt bringt.

2. Verfahren nach Anspruch 1, bei dem es sich um ein Biobegasungsverfahren handelt.

3. Verfahren nach Anspruch 1 oder Anspruch 2, wobei das Glucosinolat und die Myrosinase unter Bildung eines Isothiocyanats in Wechselwirkung treten.

4. Verfahren nach Anspruch 3, wobei das Isothiocyanat aus Benzylisothiocyanat, Isopropylisothiocyanat, Methylisothiocyanat, 1-Naphthylisothiocyanat, 2-Phenylethylisothiocyanat, Phenylisothiocyanat und Propylisothiocyanat ausgewählt ist.

5. Verfahren nach einem der vorhergehenden Ansprüche, wobei die Glucosinolat sezernierende Pflanze aus der Ordnung Brassicales stammt.

6. Verfahren nach einem der vorhergehenden Ansprüche, bei dem es sich um ein Verfahren zur Bekämpfung einer pathogenen Infektion in dem Erdboden handelt.

7. Verfahren nach Anspruch 6, bei dem es sich um ein Verfahren zur Bekämpfung pathogener Nematoden handelt.

8. Verwendung einer Kombination einer Myrosinase sezernierenden *Cryptococcus-Hefe* oder einer Spore davon und einer Glucosinolat sezernierenden Pflanze oder einem Samen davon zur Behandlung von Erdboden.

9. Kit zur Behandlung von Erdboden, wobei das Kit eine Glucosinolat sezernierende Pflanze oder einen Samen davon und eine Myrosinase sezernierende *Cryptococcus-Hefe* oder eine Spore davon umfasst.

10. Verwendung einer Myrosinase sezernierenden *Cryptococcus-Hefe* zur Behandlung von Erdboden, wobei der Erdboden eine Glucosinolatquelle umfasst.

11. Samen einer Glucosinolat sezernierenden Pflanze, der eine Beschichtung umfasst, wobei die Beschichtung eine Myrosinase sezernierende *Cryptococcus-Hefe* oder eine Quelle davon umfasst.

12. Verfahren, Verwendung, Kit oder Samen nach einem der vorhergehenden Ansprüche, wobei die Myrosinase sezernierende Hefe aus *Cryptococcus macerans* und *Cryptococcus oierensis* ausgewählt ist.

13. Verfahren, Verwendung, Kit oder Samen nach einem der vorhergehenden Ansprüche, wobei die Myrosinase sezernierende Hefe die SEQUENZ ID 1 oder die SEQUENZ ID 2 aufweist.

## Revendications

1. Méthode de traitement d'un sol, la méthode comprenant la mise à disposition vis-à-vis du sol d'une plante sécrétant du glucosinolate ou d'une semence de celle-ci et la mise en contact dudit sol avec une levure du genre *Cryptococcus* sécrétant de la myrosinase, ou une spore de celle-ci.

2. Méthode selon la revendication 1, qui est une méthode de biofumigation.

3. Méthode selon la revendication 1 ou la revendication 2, dans laquelle le glucosinolate et la myrosinase interagissent afin de former un isothiocyanate.

4. Méthode selon la revendication 3, dans laquelle l'isothiocyanate est choisi parmi l'isothiocyanate de benzyle, l'isothiocyanate d'isopropyle, l'isothiocyanate de méthyle, l'isothiocyanate de 1-naphtyle, l'isothiocyanate de 2-phényléthyle, l'isothiocyanate de phényle et l'isothiocyanate de propyle.

5. Méthode selon l'une quelconque des revendications précédentes, dans laquelle la plante sécrétant du glucosinolate appartient à l'ordre des Brassicales.

6. Méthode selon l'une quelconque des revendications précédentes, qui est une méthode de lutte contre une infection pathogène dans le sol.

7. Méthode selon la revendication 6, qui est une méthode de lutte contre des nématodes pathogènes.

8. Utilisation d'une combinaison d'une levure *Cryptococcus* sécrétant de la myrosinase, ou d'une spore de celle-ci, et d'une plante sécrétant du glucosinolate, ou d'une semence de celle-ci, pour le traitement d'un sol.

9. Kit de traitement d'un sol, le kit comprenant une plante sécrétant du glucosinolate, ou une semence de celle-ci, et une levure *Cryptococcus* sécrétant de la myrosinase, ou une spore de celle-ci.

10. Utilisation d'une levure *Cryptococcus* sécrétant de la myrosinase pour le traitement d'un sol, dans laquelle le sol comprend une source de glucosinolates.

11. Semence d'une plante sécrétant du glucosinolate comprenant un enrobage, où l'enrobage comprend une levure *Cryptococcus* sécrétant de la myrosinase, ou une source de celle-ci.

12. Méthode, utilisation, kit ou semence selon l'une quelconque des revendications précédentes, la levure sécrétant de la myrosinase étant choisie parmi *Cryptococcus macerans* et *Cryptococcus oierensis.*

13. Méthode, utilisation, kit ou semence selon l'une quelconque des revendications précédentes, la levure sécrétant de la myrosinase présentant la SEQUENCE ID n°1 ou la SEQUENCE ID n°2.
